# EUROPEAN PATENT APPLICATION

(11) **EP 1 952 765 A1**
(43) Date of publication of application: **06.08.2008**
(21) Application number: 08250335.0
(22) Date of filing: 29.01.2008
(51) Int. Cl.: A61B 5/107, G01G 19/50

(54) **Apparatus and method for determining obesity-related disease risk indicators of a human subject**

(30) Priority: 31.01.2007 US 898761 P; 10.07.2007 US 827076; 07.09.2007 US 899901
(71) Applicant: Fook Tin Technologies Ltd., Quarry Bay (HK)
(72) Inventor: Chai, John Y., Jardines Lookout (HK)
(74) Representative: Hackett, Sean James

(57) **Abstract**

A measurement device and method that in one aspect provides a simple-to-use measurement device that determines and displays obesity-related disease (metabolic syndrome) risk indicators that are calculated based on anthropometric measurements and additional information such as the subject's age and/or gender. The device may also provide classification information, such as whether the risk level is considered to be below normal, normal, increased, high, very high or extremely high.

## Description

### RELATED APPLICATION

This application is a continuation-in-part of Ser. No. 11/827,076, filed July 10, 2007, which claims priority to Provisional Application No. 60/898,761, filed January 31, 2007, each of which is incorporated herein by reference.

### BACKGROUND OF THE INVENTION

Anthropometric measurements such as height, weight, body length and head circumference have been used by medical professionals as parameters to monitor the growth of a baby, child or adolescent. Such parameters vary according to the age of the subject. Through population studies, population percentiles of such parameters have been established. It is common practice for pediatricians to measure those parameters for their patients and plot the results on respective growth charts so that the growth status of a child for each of the parameters can be determined. The apparatuses used to measure such parameters include tape measures, standing stadiometers and weighing scales.

Davignon described in CA 2,133,156A and US 5,763,837 how to improve the recording of weight and height, and estimating weight and height growth percentiles. Through the use of a weight and height measurement system, the measured weight and height of the subject is compared to growth height and weight tables relative to the sex and age category of the subject. The system then calculates the percentile weight and height of the subject from these tables.

It is also known from the medical literature that the weight and height of a child is not, by themselves, as indicative of the subject's growth pattern as certain growth indices. Examples of such growth indices are Body Mass Index (BMI) for children and adolescents, which is represented as body mass (kg) divided by the square of body height (m²), weight-for-length for infants, and weight-for-height for preschoolers. While current weight scales and weight/height measurement devices provide BMI results or the results of other indices (WO 98/13674, US 2002/00049546A1, JP2002-165764, JP2001-091346), they do not, to the inventor's knowledge, determine and display percentile information relating to growth indices based on the subject's age and gender. They also do not characterize that information in terms of whether it is considered to be, for example, below normal, normal, above normal, or far above normal.

There is accordingly a need for a measurement device that determines and displays percentile information relating to growth indices, such as BMI, weight-for-length, and weight-for-height, based on the subject's age and gender. There is also a need for a measurement device that readily provides classification information, such as whether the percentile information is considered to below normal, normal, above normal, or far above normal.

Alternatively, anthropometric measurements such as height, weight, waist or hip circumference have been used to compile mathematical ratios as indicators for management of obesity-related diseases. Medical conditions such as stroke, diabetes and coronary heart disease are significantly associated with obesity. Patients who suffer from or are at risk of suffering from these diseases commonly present a similar clinical picture of related clinical and biochemical disorders, including central adiposity, elevated triglycerides and low HDL-C (atherogenic dyslipidemia), elevated blood pressure, Insulin resistance, with or without glucose intolerance, and altered level of prothrombotic and proinflammatory markers (increased c-reactive protein, fibrinogen, and other coagulation factors). The presentation of these disorders together has been called the metabolic syndrome. (Grundy SM, "Definition of Metabolic Syndrome," Circulation, 2004;109;433-438). Metabolic syndrome is highly prevalent among Americans. Data obtained from the Third National Health and Nutrition Examination Survey suggested that the prevalence of metabolic syndrome increased from under 7% in the 20 to 29 year age group to 43.5% among those aged 60 to 69 years. (Ford ES et al.,, "Prevalence of the Metabolic Syndrome Among US Adults -- Findings from the Third National Health and Nutrition Examination Survey," JAMA, 2002;287:3;356-359). Furthermore, subjects suffering from metabolic syndrome are more likely to be associated with all-cause mortality or cardiovascular disease mortality. Proper medical assessment and management of metabolic syndrome is needed in the prevention and management of obesity-related diseases.

Obesity-related disease (metabolic syndrome) risk indicators derived from anthropometric measurement provide an easy screening tool for the public and healthcare professionals to identify subjects with increased risk so that prompt medical attention may be given. The efficacy of these indicators is well established in the literature. For example, Ashwell found that the ratio of waist circumference to height is a better indicator of coronary heart disease than body mass index (weight/height²) or waist circumference in both genders. (Ashwell M, "Ratio of waist circumference to height may be better indicator of need for weight management," BMJ 1996;312(7027):377). Also, waist-to-height ratio was found to be a better indicator than body mass index or waist circumference alone to indicate increased cardiovascular risks in non-obese Japanese men and women. (Hsieh SD et al., "The superiority of waist-to-height ratio as an anthropometric index to evaluate clustering of coronary risk factors among non-obese men and women," Preventative Medicine 2005;40:216-220). Another study showed that visceral (intra-abdominal) fat, which commonly expresses as central obesity, itself a component of the metabolic syndrome, was better predicted by waist circumference than BMI or waist-hip ratio (Valsamakis G et al., "Association of simple anthropometric measures of obesity with visceral fat and the metabolic syndrome in male Caucasian and Indo-Asian subjects," Diabetic Medicine 2004;21:1339-1345).

Dumomay et al. described in US 7,170,016 B2 a body scale capable of calculating and displaying a subject's body mass related risk factor. The so-called body mass risk factor is the difference between the subject's body mass and his or her ideal body mass. That information, however, does not in itself contribute to an understanding of the subject's risk of obesity-related disease (metabolic syndrome).

Hall et al. in US 2007/0068539A1 describes an Internet-based health management system comprised of a monitoring device configured to collect blood test information, such as total cholesterol, glucose and insulin; vital sign information, such as heart rate, blood pressure, pulse oximetry, temperature, weight and percent body fat; and the subject's own information. The information is then transmitted through the Internet to a central computer, which processes the information, and determines and indicates if the patient has metabolic syndrome. Such a system requires the user to have computer knowledge and Internet access. Its hardware interface between monitoring devices and the central computer is complex. Its user interface is based on one or more web pages. It requires multiple inputs of blood test and vital sign information for the assessment of whether a patient has metabolic syndrome. Furthermore, the system is designed for the diagnosis of metabolic syndrome rather than assessing and displaying obesity-related disease (metabolic syndrome) risk indicators.

Omron^{™} model HBF 500 body composition monitor with scale (ONIRON HEALTHCARE, INC. 1200 Lakeside Drive, Bannockburn, Illinois 60015) incorporates a feature of estimating visceral fat of the body and displaying the relative amount on a numeric scale of 30 levels. A visceral fat level at or below 9 is regarded normal and one at or above 10 is regarded high. Both visceral fat level and its classification of normal or high are displayed on the device's LCD. Similarly, Tanita UK Ltd. (The Barn, Philpots Close, Yiewsley, Middlesex, UB7 7RY U K) also markets certain body composition monitors such as model BC-533, which possesses features such as visceral fat estimation, its rating display between 1 and 59 and classification between "healthy" and "excess" levels. It appears that visceral fat or abdominal fat, itself not an anthropometric measurement, is estimated by these devices using bioelectrical impedance analysis. Although excess visceral fat may contribute to metabolic syndrome, the display of the relative amount and classification of visceral fat does not help users easily understand their risk of suffering from obesity-related disease (metabolic syndrome).

There is accordingly a need for a simple-to-use measurement device that determines and displays obesity-related disease (metabolic syndrome) risk indicators that are calculated based on anthropometric measurements and additional information such as the subject's age and gender. There is also a need for a measurement device that readily provides classification information, such as whether the risk level is considered to be below normal, normal, increased, high, very high or extremely high.

### SUMMARY OF THE INVENTION

In one aspect, the present invention provides a measurement device that determines and displays percentile information relating to growth indices, such as BMI, weight-for-length, and weight-for-height, based on a subject's age and gender.

In another aspect, the present invention provides classification information, such as whether the percentile information is considered to be, for example, below normal, normal, above normal, or far above normal. For example, in one embodiment, a measurement device in accordance with the present invention may indicate whether the BMI-for-age percentile, for ages 2-18, is in one of the following four ranges: underweight, normal, risk of overweight, and overweight.

To assist the subject in understanding his or her normal growth profile, another embodiment of the measurement device may additionally indicate, for example, the normal range of the subject's weight based on the subject's height, the subject's height based on the subject's weight and the normal range of BMI based on the subject's age and gender.

In yet another embodiment, the measurement device may also indicate predictive values of the subject's future weight, height, BMI or head circumference based on the subject's present measurement values and growth percentiles. Such an embodiment helps the subject in understanding his or her growth trend.

In another aspect, the present invention provides a simple-to-use measurement device that determines and displays obesity-related disease (metabolic syndrome) risk indicators that are calculated based on anthropometric measurements and additional information such as the subject's age and gender. In yet another aspect, the present invention may provide classification information for an obesity-related disease (metabolic syndrome) risk indicator, such as whether the risk level is considered to be below normal, normal, increased, high, very high or extremely high.

### BRIEF DESCRIPTION OF THE DRAWINGS

Additional objects and features of the invention will be more readily apparent from the following detailed description and appended claims when taken in conjunction with the drawings, in which:

FIG. 1 is a block diagram of a measurement device in accordance with the present invention;

FIG. 2 is a flow chart illustrating the operation of a measurement device in accordance with the present invention;

FIG. 3 is an example of a body weight scale in accordance with the present invention;

FIG. 4 depicts an exemplary LCD display in accordance with the present invention.

FIG. 5 shows a sequence of display screens during measurement of a male child or adolescent.

FIG. 6 shows a sequence of display screens during measurement of an adult male.

FIG. 7 depicts an exemplary LCD display for displaying obesity-related disease (metabolic syndrome) risk indicator classification information.

FIG. 8 shows the display of "increased" obesity-related disease risk.

Like reference numerals refer to corresponding parts throughout the several views of the drawings.

### DETAILED DESCRIPTION OF THE PREFERRED EMBODIMENTS

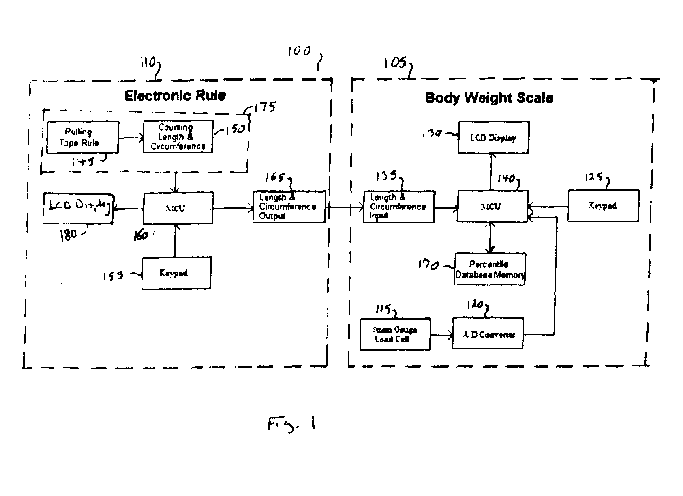

FIG. 1 is a block diagram of a measurement device 100 in accordance with the present invention. Device 100 includes a body weight scale 105 and an optional electronic rule 110.

Body weight scale 105 includes strain gauge load cell 115, A/D converter 120, keypad 125, LCD display 130, optional length & circumference input 135, micro control unit (MCU) 140, and percentile database memory 170. Strain gauge load cell 115 produces an analog signal corresponding to and indicative of the weight of a subject positioned on it, as is well known in the art. That analog signal is converted into a digital signal by A/D converter 120 and then input to MCU 140. Alternatively, instead of strain gauge load cell 115 and/or A/D converter 120, any suitable weight measurement system that produces a digital signal corresponding to the weight of a subject may be used. MCU 140 can be any type of digital device capable of performing the operations described below of a measurement device in accordance with the present invention, including, but not limited to, a microcontroller, microprocessor, programmable logic array, or application specific integrated circuit (ASIC). Keypad 125 is used to enter additional information into MCU 140, such as the subject's age, sex, and, optionally, length/height and, in certain embodiments, head circumference. Alternatively, length/height and/or head circumference information may be entered through length & circumference input 135, which in turn may receive that information from electronic rule 110, described below. LCD display 130 displays various information input into or determined by MCU 140. Percentile database memory 170 contains percentile information for various growth indices based on age and gender. It may also contain other information that may be used in embodiments of the present invention. Various electronic components of body weight scale 105 are powered by a battery or other power supply, not shown.

Electronic rule 110, if present, may be any device that electronically measures a subject's length or height and/or the circumference of a subject's head or waist. Such devices are well known in the art. In one embodiment, electronic rule 110 includes tape rule or height measurement device 175, MCU 160, keypad 155, LCD display 180 and length and circumference output 165.

Tape rule or height measurement device 175 may be any suitable device known in the art for electronically determining the length or height of a subject or the circumference of a subject's head, waist or hip. It may include, for example, a pulling tape or rod rule 145 and a counting length and circumference unit 150. Pulling tape or rod rule 145 may comprise a reel of soft tape or a height rod having holes distributed at known intervals along its length through which light emitted from an LED may pass. Counting length and circumference unit 150 detects and counts the pulses as the tape or rod is advanced with increasing length or height and uses the count to determine the measured length/height of the subject and/or the circumference of a part. of the subject's body, such as the head, waist or hip. The length/height information and/or circumference is input into MCU 160, which in turn sends it, via length and circumference output 165, to the length and circumference input 135 of body weight scale 105. Various electronic components of electronic rule 110 are likewise powered by a battery or other power supply, not shown.

The various components depicted in Figure 1 may be connected, or linked, via wire and/or other physical components and/or via a wireless connection, such as infrared transmission, as known in the art. For example, display 130 and keypad 125 may be contained in a separate housing from the other components of body weight scale 105, including MCU 140, and communicate with MCU 140 via infrared transmission, as known in the art.

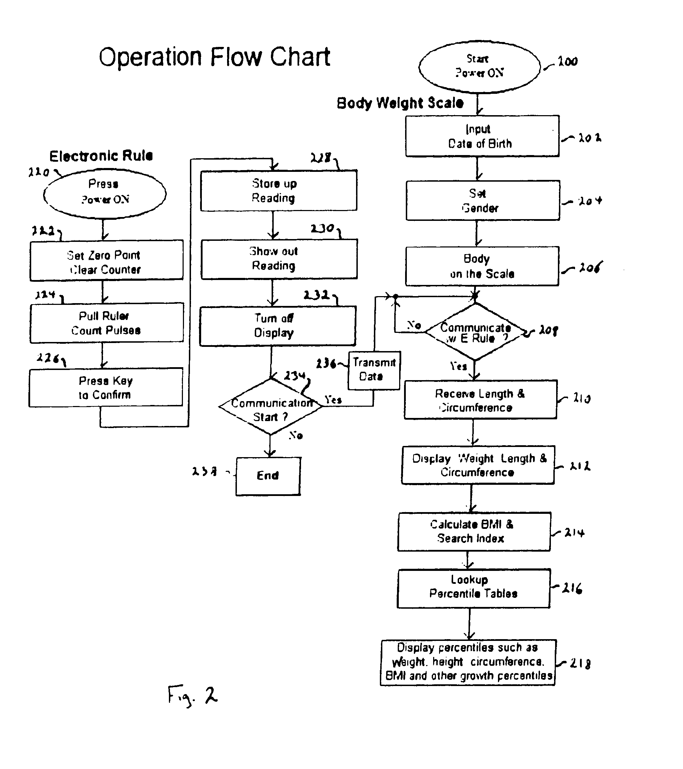

FIG. 2 is a flow chart illustrating the operation of a measurement device in accordance with the present invention. Steps 200 through 218 relate to the operation of body weight scale 105. Steps 220 through 238 relate to the operation of electronic rule 110, if present.

In step 200, body weight scale 105 is powered on. In step 202, the subject's date of birth is entered into MCU 140 and, in step 204, the subject's gender is entered into MCU 140, both via keypad 128. In step 206, the subject is weighed on strain gauge load cell 115, causing the subject's weight to be entered into MCU 140 via A/D converter 120. Alternatively, instead of strain gauge load cell 115 and A/D converter 120, other suitable weight measuring components or devices may be used that are capable of generating a digital signal corresponding to a subject's weight. In step 208, MCU 140 attempts to communicate with electronic rule 110, and, if successful, receives length and/or height and, optionally, circumference information in step 210. Alternatively, steps 208 and 210 can be eliminated and the length/height and/or circumference information can instead be entered into MCU 140 via keypad 125. In step 212, the weight, length/height and/or optional circumference information may be displayed on display 130.

In step 214, MCU 140 calculates the subject's BMI, or other growth index. BMI is equal to weight (kg) ÷ (stature (cm))² × 10,000 or weight (1b) ÷ (stature (in))² x 703. In step 216, MCU 140 then retrieves corresponding percentile information, including BMI or other growth index percentile information, from percentile database memory 170, based on the BMI, or other growth index, and the subject's age and gender. MCU 140 may also retrieve corresponding percentile information for the subject's weight, height and/or circumference, or other growth percentiles. Alternatively, percentile information can also be derived from formulas, where known in the art. In step 218, the percentile information, including the BMI or other growth index percentile, and weight, height, circumference, and/or other growth percentiles may be displayed on display 130. In addition, classification information indicating whether the displayed measurement, index, or percentile information is considered to below normal, normal, above normal, or far above normal, may also be displayed.

In embodiments equipped with a "normal range" function, the range of normal values tor a given growth measurement, based on the subject's age, gender and/or other growth measurements, may also be displayed. For example, the normal range value of height, weight, head circumference or BMI of a 5-year-old male child may be displayed. Similarly, the normal range value of weight for a given length or height of an infant or child may be displayed.

In embodiments including the growth trend feature, MCU 140 may retrieve from percentile database memory 170 growth values and indices, such as height, weight, head circumference or BMI corresponding to the subject's future age based on the subject's present percentile. For example, given the height of a 12-year-old male subject's fits into the 50th percentile of the population, MCU 140 retrieves the 50th percentile height value of a male subject at, for example, 17 years old and displays such a predictive value on display 130.

In embodiments including electronic rule 110, in step 220, electronic rule 110 is powered on. In step 222, electronic rule 110 is cleared to zero if necessary. In step 224, tape rule 145 is pulled and the resulting pulses are counted by counting length and circumference unit 150. In step 226, when the desired length is reached, a key is pressed to confirm that measurement is complete. In step 228, the measurement is then stored in MCU 160 and, in step 230, displayed on LCD display 180, if present. If display 180 is not present, the measurement may be displayed on LCD display 130, in which case a suitable connection must be provided between MCU 160 and LCD display 130. In step 232, the display is turned off after a predetermined period of time.

In step 234, MCU 160 in electronic rule 110 waits for a communication request from MCU 140 in body weight scale 105. If a communication request is received, MCU 160 transmits, in step 236, the measurement information, via length and circumference output 165, to the length and circumference input 135 in body weight scale 105. If no communication request is received or a communication is otherwise not initiated, the process ends in step 238.

For Caucasian adults, a BMI over 30 is considered obese; between 25 and 29.9 is considered overweight, between 18.5 and 24.9 is considered normal (a healthy BMI); and under 18.5 is considered underweight. These ranges may differ for different races.

For children and adolescents, ages 2 to 18, body fatness changes with age and is gender dependent. A BMI in the 95^{th} percentile or greater is considered overweight; in the 85^{th} to 95^{th} percentile is considered at risk of being overweight; in the 5^{th} to 85^{th} percentile is considered to be normal; and less than the 5^{th} percentile is considered to be underweight.

The percentile information that is stored in percentile database memory is publicly available from, for example, the National Center for Health Statistics (which is part of the U.S. Department Of Health And Human Services' Centers for Disease Control and Prevention).

An exemplary embodiment of a measurement device in accordance with the present invention includes: high precision strain gauge technology for weight measurement; a weighing function; BMI calculation; BMI-for-age Percentile, which displays percentile for children and adolescents (2-18 years of age) according to age and gender; Classification of BMI-for-age Percentile (2-18 years of age) according to four categories: underweight, normal, risk of overweight and overweight; Classification of BMI for adults according to four categories: underweight, normal, overweight and obese; 3-button operation; 4-user memories for input data (age, gender, and, optionally, height), which stores information for four users; fast recall function; memory clear function, which clears some or all of the information in the user memories; auto-on and auto-off function, which automatically turn the measurement device on when the subject is placed on the weighing scale and off a few seconds after the subject is removed from the weighing scale in order to save power; power saving LCD readout; low-battery indicator; 150kg or 3301b weight capacity; 100g or .21b graduation; age range from 2 to 99 years; height range from 100cm to 220cm (3'3.4" to 7'2.6"); and two CR2032 lithium batteries.

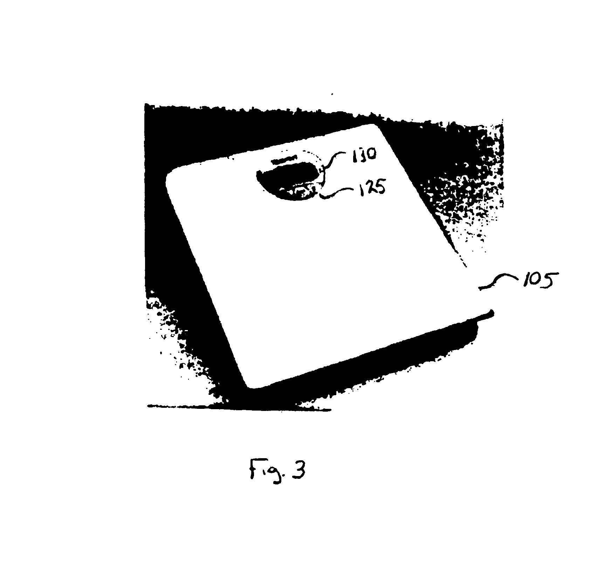

An example of a body weight scale 105, having a 3-button keypad 125 and an LCD display 130 is shown in Fig. 3.

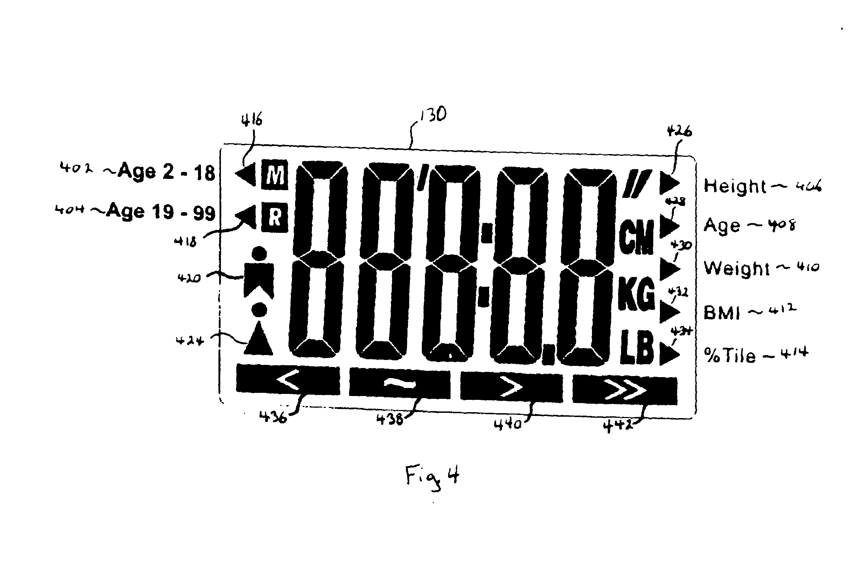

Fig. 4 depicts an exemplary LCD display 130 in which all display segments are illuminated for explanatory purposes. Indicia 402, 404, 406, 408, 410, 412, and 414 are printed on the surface adjacent to display 130. Indicator 416 is illuminated if the age of the subject, as determined by the entered birthdate, is between 2 and 18. Indicator 418 is illuminated if the age of the subject is between 19 and 99. Indicator 420 is illuminated if the subject is male and indicator 424 is illuminated if the subject is female, again as determined from the entered gender information. Indicator 426 is illuminated if the height is currently being displayed; indicator 428 is illuminated if the age is currently being displayed; indicator 430 is illuminated if the weight is currently being displayed; indicator 432 is illuminated if the BMI is currently being displayed; and indicator 434 is illuminated if the BMI percentile is currently being displayed. Indicator 436 is illuminated if the subject's BMI percentile indicates that the subject is underweight; indicator 438 is illuminated if the subject's BMI percentile indicates that the subject is normal; indicator 440 is illuminated if the subject's BMI percentile indicates that the subject is overweight (for adults) or at risk of becoming overweight (for children and adolescents); and indicator 442 is illuminated if the subject's BMI percentile indicates that the subject is obese (for adults) or overweight (for children and adolescents). Indicators 436, 438, 440 and 442 more generally indicate whether the value being displayed is considered to be below normal, normal, above normal, or far above normal. For example, in certain embodiments, head circumference-for-age, height-for-age, and/or weight-for-age percentiles may be displayed, in which case indicators 436, 438, 440 and 442 would indicate whether those values are below normal, normal, above normal, or far above normal. Similarly, weight-for-length, and/or weight-for-height percentiles may be displayed in which case indicators 436, 438, 440 and 442 would likewise indicate the classification of those percentiles. Other portions of display 130 display the various values mentioned above, and other values, in appropriate units.

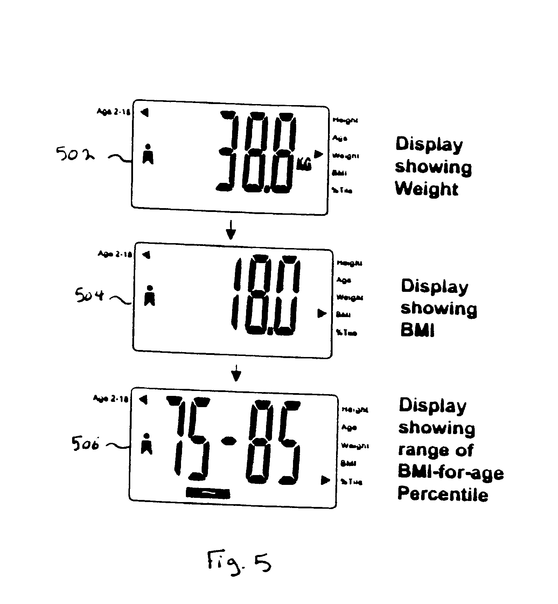

Fig. 5 shows a sequence of display screens during measurement of a male child or adolescent. Display screen 502 indicates that the subject is 38.8 kg. Display screen 504 indicates that the subject has a BMI of 18.0. Display screen 506 indicates that the subject has a BMI percentile of 75-85%. The illuminated indicator on the bottom of display screen 506 indicates that the subject's BMI percentile is in the normal range.

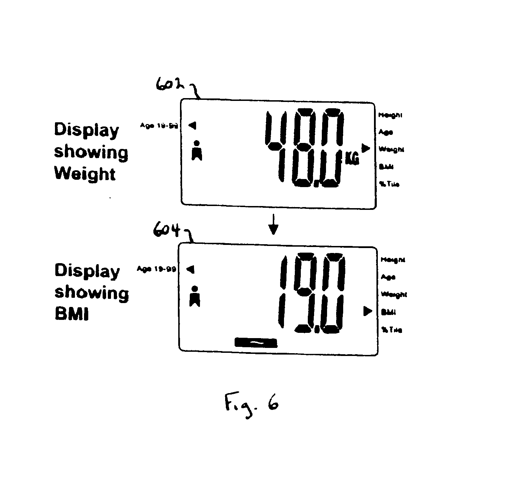

Fig. 6 shows a sequence of display screens during measurement of an adult male. Display screen 602 indicates that the subject is 48.0 kg. Display screen 604 indicates that the subject has a BMI of 19.0 and that that BMI is in the normal range.

In embodiments including an obesity-related disease (metabolic syndrome) risk indicator feature, anthropometric measurements can be entered using electronic rule 110 and/or another anthropometric measurement system. Alternatively, anthropometric measurements, as well as other information about a subject, can be entered using keypad 120. Anthropometric measurements that may be used in determining obesity-related disease (metabolic syndrome) risk indicators include, but are not limited to, height, weight, waist circumference and hip circumference. The MCU 140, in turn, uses the anthropometric measurements, as well as possibly other information about the subject, to determine obesity-related disease (metabolic syndrome) risk indicators. In one embodiment, the anthropometric measurements comprise the circumference of the subject's waist and the subject's height and the anthropometric risk indicator is the waist circumference to height ratio. The anthropometric risk indicator may also be based on, for example, a subject's BMI and waist circumference, or waist circumference to hip circumference, though the invention is not limited to a particular anthropometric risk indicator. The obesity-related disease (metabolic syndrome) risk indicator may be displayed, for example, on display 130.

An embodiment of a device in accordance with the present invention may also determine and display classification information regarding an obesity-related disease (metabolic syndrome) risk indicator. The classification information may be, for example, whether the risk level associated with a risk indicator is, for a particular age and/or gender, considered to be below normal, normal, increased, high, very high or extremely high. Other classification information may also be provided. The classification information may also depend on other factors, such as race. In such an embodiment, percentile database memory 170 may contain classification information for one or more risk indicators based on, for example, age and/or gender. The classification information can also be derived from formulas, where known in the art. The display may be the same as or incorporated into or part of display 130. Alternatively, the classification information may be displayed on a display that is separate from display 130.

For example, in an embodiment in which the obesity-related disease (metabolic syndrome) risk indicator is based on a subject's BMI and waist circumference, classification information for the obesity-related disease (metabolic syndrome) risk indicator is available from the National Heart, Lung, and Blood Institute at littp://www.nhlbi.nih.gov/health/public/heart/obesity/lose_wt/bmi_dis.htm. The National Heart, Lung, and Blood Institute is a division of the National Institutes of Health, which in turn is an Agency of the United States Department of Health and Human Services. For the waist circumference to height risk indicator, classification information is available from Ashwell Assoicates (Ashwell Street, Ashwell, Hertfordshire UK) at http://www.ashwell.uk.com/shapechart.pdf. For the waist circumference to hip circumference indicator, classification information is provided in Lin W-Y, "Optimal cut-off value for obesity: using simple anthropometric indices to predict cardiovascular risk factors in Taiwan," International Jounal of Obesity, 26, 1232-38 (2002) (giving the optimal cut-off value for the waist to hip ratio as .85 for men and .76 for women).

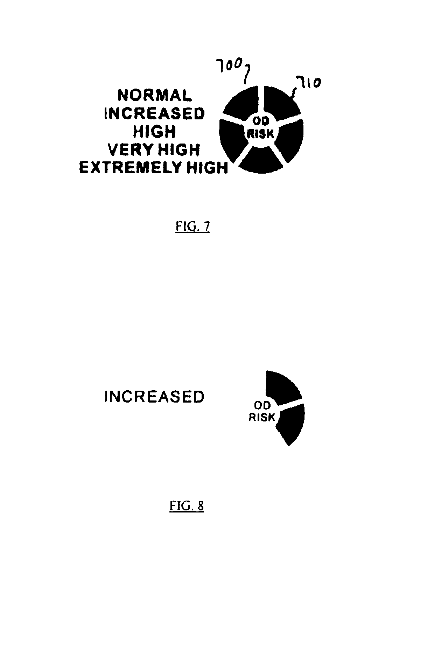

FIG. 7 depicts an exemplary LCD display 700 for displaying obesity-related disease (metabolic syndrome) risk indicator classification information. As shown, all five segments 710 of LCD display 700 are illuminated. In operation, in this embodiment, one illuminated segment indicates a normal classification; two illuminated segments indicate an increased risk classification; three illuminated segments indicate a high risk classification; four illuminated segments indicate a very high risk classification; and five illuminated segments indicate an extremely high risk classification. FIG. 8 depicts an exemplary LCD display 700 in which an increased risk classification is displayed.

The illustrative descriptions of the application of the principles of the present invention are to enable any person skilled in the art to make or use the disclosed invention. These descriptions are susceptible to numerous modifications and alternative arrangements by those skilled in the art. Such modifications and alternative arrangements are not intended to be outside the scope of the present invention. The appended claims are intended to cover such modifications and arrangements. Thus, the present invention should not be limited to the described illustrative embodiments but, instead, is to be accorded the broadest scope consistent with the principles and novel features disclosed herein.

## Claims

1. A device for providing obesity-related disease risk indicator information comprising:
a weight measurement system;
an input device; and
a micro control unit linked to the weight measurement system and the input device; and wherein the micro control unit is capable of determining an obesity-related disease risk indicator based on weight information generated by the weight measurement system and user information input on the input device.

2. A device for providing obesity-related disease risk indicator classification information comprising:
a weight measurement system;
an input device;
a memory device; and
a micro control unit linked to the weight measurement system, the input device and the memory device;
wherein the memory device stores classification information for one or more obesity-related disease risk indicators; and
wherein the micro control unit is capable of determining an obesity-related disease risk indicator classification based on the classification information stored in the memory device and one or more of user information input on the input device or weight information generated by the weight measurement system.

3. The device of claim 1 or claim 2 further comprising:
an anthropometric measurement system capable of taking anthropometric measurements of a subject other than weight; and
wherein the micro control unit is further linked to the anthropometric measurement system; and
the obesity-related disease risk indicator or indicator classification is further based on the anthropometric information generated by the anthropometric measurement system.

4. The device of claim 1, claim 2 or claim 3 further comprising:
a display linked to the micro control unit capable of displaying information relating to the obesity-related disease risk indicator or indicator classification.

5. The device of any one of claims 1 to 4 wherein the obesity-related disease risk indicator or indicator classification comprises, or is based on, waist circumference to height information.

6. The device of any one of claims 2 to 5 wherein the classification information includes one or more of normal risk, increased risk, high risk, very high risk, or extremely high risk.

7. The device of any one of the preceding claims wherein the user information includes one or more of age information, gender information, length or height information, waist circumference information or hip circumference information.

8. The device of any one of claims 3 to 7 wherein the anthropometric measurement systems comprises an electronic rule.

9. The device of claim 8 wherein the electronic rule comprises a pulling tape or rod rule and a counting length or circumference unit.

10. The device of any one of claims 1 to 9 wherein
the micro control unit is capable of determining a body mass index of a subject from weight information generated by the weight measurement system and height information input on the input device; and
the obesity-related disease risk indicator or indicator classification is based on waist circumference and body mass index.

11. The device of claim 3 wherein the anthropometric measurements are one or more of height, waist circumference or hip circumference.

12. The device of claim 1 or claim 2 wherein the obesity-related disease risk indicator or indicator classification comprises, or is based on, waist circumference to hip circumference information.

13. A method for providing obesity-related disease risk indicator information comprising:
receiving weight information from a weight measurement system linked to a micro control unit;
receiving user information from an input device linked to the micro control unit; and
determining an obesity-related disease risk indicator based on the weight information and user information.

14. A method for providing obesity-related disease risk indicator classification information comprising:
receiving one or more of user information input on an input device linked to a micro control unit or weight information received from a weight measurement system linked to the micro control unit;
determining an obesity-related risk indicator classification based on classification information for one or more obesity-related disease risk indicators stored in a memory device and the received one or more user information and weight information.

15. The method according to claim 13 or claim 14 further comprising:
receiving anthropometric measurements of a subject other than weight from an anthropometric measurement system linked to the micro control unit; wherein
the obesity-related indicator or indicator classification is further based on the anthropometric information generated by the anthropometric measurement system.

16. The method according to claim 13, 14 or 15 further comprising:
displaying information relating to the obesity-related disease risk indicator or indicator classification on a display linked to the micro control unit.

17. The method according to any one of claims 13 to 16 wherein the obesity-related disease risk indicator or indicator classification comprises, or is based on, waist circumference to height information.

18. The method according to any one of claims 13 to 17 wherein the user information includes one or more of age information, gender information, length or height information, waist circumference information or hip circumference information.

19. The method according to any one of claims 13 to 18 wherein the user information comprises height information and waist circumference information; and further comprising the step of:
determining a body mass index of a subject from the weight information and the height information, wherein
the obesity-related risk indicator or indicator classification is based on waist circumference and body mass index.

20. The method according to any one of claims 15 to 19 wherein the anthropometric measurements are one or more of height, waist circumference or hip circumference.

21. The method according to any one of claims 13 to 20 wherein the obesity-related disease risk indicator or indicator classification comprises, or is based on, waist circumference to hip circumference information.

22. The method according to claim 14 or any of claims 15 to 21 when dependent from claim 14 wherein the classification information includes one or more of normal risk, increased risk, high risk, very high risk or extremely high risk.
